# EUROPEAN PATENT APPLICATION

(11) **EP 4 772 629 A1**
(43) Date of publication of application: **08.07.2026**
(21) Application number: 23955861.2
(22) Date of filing: 31.10.2023
(51) Int. Cl.: C12N 15/11, C12N 15/10, C12N 9/00

(54) **RNA CONTAINING PHOSPHOROTHIOATE BOND, AND PREPARATION METHOD THEREOF**

(30) Priority: 16.10.2023 CN 202311335112
(71) Applicant: Jilin Asymchem Laboratories Co., Ltd., Dunhua, Jilin 133700 (CN); Tianjin Asymchem Pharmaceuticals Co., Ltd, Tianjin 300457 (CN)
(72) Inventor: HONG, Hao, Morrisville, NC 27560 (US); JAMES, Gage, Morrisville, NC 27560 (US); ZHANG, Na, Tianjin 300457 (CN); JIAO, Xuecheng, Tianjin 300457 (CN); PANG, Huining, Tianjin 300457 (CN); LIU, Fang, Tianjin 300457 (CN); MA, Cuiping, Tianjin 300457 (CN); WANG, Zhaoshuai, Tianjin 300457 (CN); JIA, Xu, Tianjin 300457 (CN); CUI, Lixin, Tianjin 300457 (CN); ZHU, Wenxuan, Tianjin 300457 (CN); ZHAO, Xiaolan, Tianjin 300457 (CN)
(74) Representative: Zacco GmbH
(86) International application number: PCT/CN2023/128832
(87) International publication number: WO 2025/081543

(57) **Abstract**

Provided is RNA containing a phosphorothioate bond, and a preparation method thereof. The method includes: catalyzing the ligation of 3' hydroxyl of a substrate with 5' phosphorothioate with an RNA ligase, so as to form the RNA containing a phosphorothioate bond, where the 5' phosphorothioate includes 5' phosphate containing a thio modification, and the thio modification includes the replacement of an oxygen atom in P-OH or P=O of the 5' phosphate by a sulfur atom, thereby forming P-SH or P=S accordingly; and the RNA ligase includes any one or more enzymes of RNA ligase family 1, 2 or 3, specifically, one or more of RNA ligases shown in sequences such as SEQ **ID** NOs: 1-22.

## Description

This application is based upon and claims priority to Chinese Patent Application No. 202311335112.8 filed on October 16, 2023, the disclosure of which is hereby incorporated by reference in its entirety.

### Technical Field

The present disclosure relates to the field of RNA preparation, and specifically, to RNA containing a phosphorothioate bond, and a preparation method thereof.

### Background

RNA drugs are new drugs merged in recent years and classified into three categories based on their mechanisms of action: the first category is small nucleic acid drugs that target nucleic acids to inhibit or activate gene expression activity, including Antisense Oligonucleotide (ASO), small interfering RNA (siRNA), microRNA (miRNA), small activating RNA (saRNA), etc.; the second category is RNA aptamers that target proteins to regulate protein activity; and the third category is messenger RNA (mRNA) that encodes therapeutic proteins or antigens. RNA drugs have numerous advantages, such as strong targeting specificity, simple design, short development cycles, rich candidate targets, etc. Since the U.S. FDA approved the first RNA drug fomivirsen (now withdrawn from the market) in 1998, dozens of RNA drugs have entered the market to date, and more RNA drugs are currently in development and clinical research phases.

Stability chemical modifications are one of the key strategies in nucleic acid drug design. Safety and efficacy are the primary challenges in the design and development of RNA-targeting drugs, and are also the main reasons for early-stage drug development failures. The thio modification, which means that an oxygen atom in P-OH or P=O of a phosphodiester bond between nucleotides is replaced by a sulfur atom, of the phosphodiester bond in oligonucleotide has been widely applied in the development of RNA drugs. The thio modification of the phosphodiester bond in the RNA drug has a dual effect by enhancing nuclease resistance and promoting binding to plasma proteins, thereby prolonging the elimination half-life of drugs, increasing in-vivo circulation time of the drugs, and improving the pharmacokinetics of the drugs. Meanwhile, the thiol modification introduces chirality, causing two orientations to have significant different pharmacokinetic and pharmacodynamic properties, thus affecting drug development. The thio modification also reduces the affinity of oligonucleotide for targets. However, the synthesis of thio-oligonucleotide in the prior art is primarily achieved through chemical reaction preparation using thio-reagents during solid-phase synthesis, and there are currently no studies on the preparation of RNA containing a phosphorothioate bond using biological methods such as enzyme catalysis, etc.

### Summary

The present disclosure is mainly intended to provide RNA containing a phosphorothioate bond, and a preparation method thereof, so as to solve the problem in the prior art of difficult preparation of RNA containing a phosphorothioate bond using an enzyme catalysis method.

In order to implement the above objective, according to a first aspect of the present disclosure, a method for preparing an RNA containing a phosphorothioate bond is provided. The preparation method includes: the ligation of 3' hydroxyl of a substrate with 5' phosphorothioate is catalyzed with an RNA ligase, so as to form the RNA containing the phosphorothioate bond, where the 5' phosphorothioate includes 5' phosphate containing a thio modification, and the thio modification includes the replacement of an oxygen atom in P-OH or P=O of the 5' phosphate by a sulfur atom, thereby forming P-SH or P=S accordingly; and the RNA ligase includes any one or more enzymes of RNA ligase family 1, 2 or 3.

Further, the RNA ligase includes one or more of RNA ligases shown in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21 or SEQ ID NO: 22; or an enzyme having more than 70%, 75%, 80%, 85%, 90%, 91%, 92%, 93%, 94%, 95%, 96%, 97%, 98%, 99%, 99.5%, or even 99.9% identity to any one of the RNA ligases shown in SEQ ID NO: 1 to SEQ ID NO: 22, and having a protein that catalyzes the activity of the ligation of the 3' hydroxyl and the 5' phosphorothioate.

Further, the substrate includes a single-stranded RNA or a double-stranded RNA, and correspondingly, the RNA containing the phosphorothioate bond includes a single-stranded RNA containing a phosphorothioate bond or a double-stranded RNA containing a phosphorothioate bond; and one, two or more kinds of substrates are provided.

Further, when one kind of substrate is provided and the substrate is the single-stranded RNA, the 5' terminal of the substrate is the 5' phosphorothioate, and the 3' terminal of the substrate is the 3' hydroxyl, and the 5' terminal of the substrate and the 3' terminal of the substrate are ligated and cyclized under the catalysis of the RNA ligase, so as to obtain a cyclic single-stranded RNA containing a phosphorothioate bond.

Further, the cyclization of the single-stranded RNA is guided using a splint, the splint specifically binds to at least three bases from the 3' terminal to the 5' terminal of the single-stranded RNA, and the splint specifically binds to at least three bases from the 5' terminal to the 3' terminal of the single-stranded RNA, and bases of the splint which specifically bind to the 3' terminal of the single-stranded RNA are adjacent to bases which specifically bind to the 5' terminal of the single-stranded RNA.

Further, when two kinds of substrates are provided and the substrates are the single-stranded RNA, the substrates include a first substrate and a second substrate, the 3' terminal of the first substrate is the 3' hydroxyl, and the 5' terminal of the second substrate is the 5' phosphorothioate; and the 3' terminal of the first substrate and the 5' terminal of the second substrate are ligated under the catalysis of the RNA ligase to obtain a linear single-stranded RNA containing a phosphorothioate bond.

Further, the 3' terminal of the second substrate is a 3' terminal protecting group, and the 5' terminal of the first substrate is a 5' terminal protecting group.

Further, when two kinds of substrates are provided and the substrates are the double-stranded RNA, each substrate includes a sense strand and an antisense strand, and the substrates include a first substrate and a second substrate; the 3' terminal of the sense strand of the first substrate is the 3' hydroxyl, and the 5' terminal of the antisense strand of the first substrate is the 5' phosphate; the 5' terminal of the sense strand of the second substrate is the 5' phosphate, and the 3' terminal of the antisense strand of the second substrate is the 3' hydroxyl; at least one of the 5' phosphate of the antisense strand of the first substrate and the sense strand of the second substrate is the 5' phosphorothioate; the 3' terminal of the sense strand of the first substrate and the 5' terminal of the sense strand of the second substrate are ligated under the catalysis of the RNA ligase; and the 5' terminal of the antisense strand of the first substrate and the 3' terminal of the antisense strand of the second substrate are ligated under the catalysis of the RNA ligase, so as to obtain linear double-stranded RNA containing a phosphorothioate bond.

Further, the 3' terminal of the antisense strand of the first substrate and the 3' terminal of the sense strand of the second substrate are both 3' terminal protecting groups; and the 5' terminal of the sense strand of the first substrate and the 5' terminal of the antisense strand of the second substrate are both 5' protecting groups.

Further, the first substrate and the second substrate are both double-stranded RNA with the lengths of a sense strand and antisense strand being different. The 3' terminal of the sense strand of the first substrate is complementarily paired with the 3' terminal of the antisense strand of the second substrate, or the 5' terminal of the antisense strand of the first substrate is complementarily paired with the 5' terminal of the sense strand of the second substrate. Thereby, the 3' terminal of the sense strand of the first substrate is adjacent to the 5' terminal of the sense strand of the second substrate, and the 5' terminal of the antisense strand of the first substrate is adjacent to the 3' terminal of the antisense strand of the second substrate.

Further, the preparation method further includes: a splint is used to guide the ligation between the first substrate and the second substrate, where the splint specifically binds to at least three bases from the 3' terminal to the 5' terminal of the first substrate, and the splint specifically binds to at least three bases from the 5' terminal to the 3' terminal of the second substrate.

Further, bases of the splint specifically binding to the 3' terminal of the first substrate are adjacent to bases specifically binding to the 5' terminal of the second substrate.

Further, the preparation method is repeatedly used to ligase three or more substrates for a plurality of times, the kind of the substrates ligated each time is two, and the substrates are sequentially ligated to obtain the RNA containing the phosphorothioate bond.

Further, the preparation method includes: a splint is used to guide the ligation of three or more kinds of substrates; and the splint specifically binds to at least three bases of each of the substrates.

Further, bases of the substrates specifically binding to the splint are adjacent to each other, and the adjacent bases are located at the 5' terminal and 3' terminal of different substrates, respectively; the 5' terminal is the 5' phosphate, and at least one of the 5' phosphate is the 5 phosphorothioate; and the 3' terminal is the hydroxyl.

Further, a catalytic system of the RNA ligase includes 1-20000 µM of the substrate, 1-100000 µM of ATP, 5-100 mM of Tris-HCl, 0.1-20 mM of bivalent metal ions, 0-10 mM of dithiothreitol, and 0.001-10 mg/mL of the RNA ligase.

Further, a catalytic temperature of the RNA ligase is 4-50 °C, and a catalytic time is 0.1-24 h; and the divalent metal ions are derived from MgCl₂ or MgSO₄.

Further, the substrate includes one or more non-natural nucleotides, and the RNA containing the phosphorothioate bond includes a non-natural RNA containing a phosphorothioate bond.

Further, the number of the non-natural nucleotides in the substrate is ≥2, and the 5' terminal and/or 3' terminal of the substrate are the non-natural nucleotides.

Further, the substrate consists of the non-natural nucleotides.

Further, the non-natural nucleotides include ribonucleotide having one or more of the following modifications: a ribose position 2' modification, a ribose backbone modification, a base modification or a phosphate backbone modification; the ribose position 2' modification includes a 2'-methoxy modification, a 2'-fluoro modification, a 2'-hydrogen modification, a 2'-methoxyethyl modification, a 2'-FANA modification, a locked nucleic acid modification or a hexitol nucleic acid modification; the ribose backbone modification includes replacing a ribose in the nucleotide with ribuloNA, TNA, tPhoNA, or dXNA; the base modification includes a deazaadenine C7 modification, a deazaguanosine C7 modification, a cytosine C5 modification, or a uridine C5 modification; and the phosphate backbone modification includes a PS modification.

In order to implement the above objective, according to a second aspect of the present disclosure, an RNA containing a phosphorothioate bond is provided. The RNA containing the phosphorothioate bond includes the RNA containing the phosphorothioate bond obtained through preparation using the above preparation method.

With the technical solutions of the present disclosure, the 3' hydroxyl of the substrate is ligated to the 5' phosphorothioate with the above preparation method and the RNA ligase, such that one substrate can be ligated to form a cyclic RNA, or the plurality of substrates are ligated to form a linear RNA, thereby obtaining, through preparation, the RNA containing the phosphorothioate bond difficultly obtained in the prior art.

### Brief Description of the Drawings

The drawings, which form a part of the present application, are used to provide a further understanding of the present disclosure. The exemplary embodiments of the present disclosure and the description thereof are used to explain the present disclosure, but do not constitute improper limitations to the present disclosure. In the drawings:

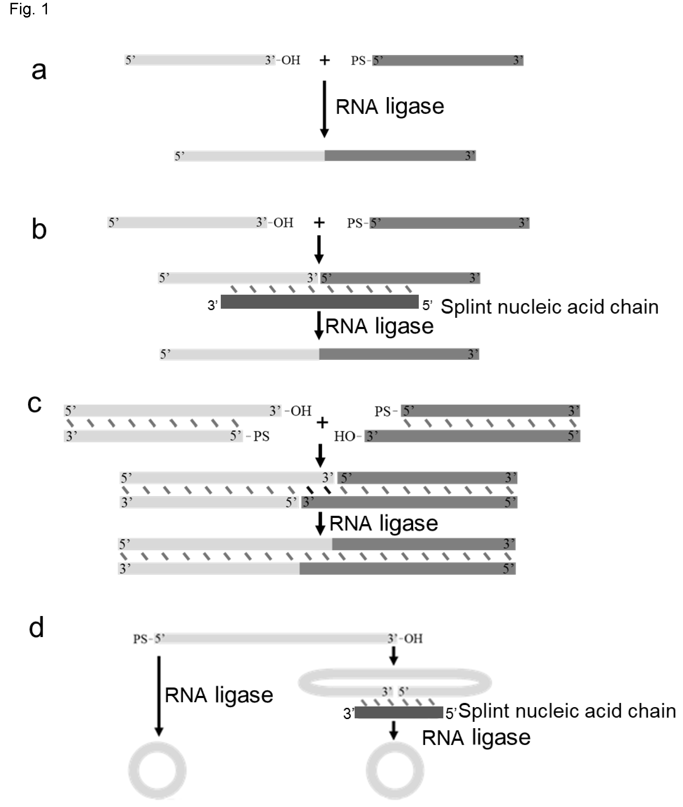

Fig. 1 is a schematic diagram of a preparation method according to embodiments of the present disclosure, where a in Fig. 1 is a schematic diagram of the ligation of two pieces of single-stranded RNA using the above method; b in Fig. 1 is a schematic diagram of the use of a splint to guide the ligation of two pieces of single-stranded RNA substrates using the above method; c in Fig. 1 is a schematic diagram of two pieces of double-stranded RNA using the above method; and d in Fig. 1 is a schematic diagram of cyclization of the substrate to form a single-stranded circular RNA using the preparation method described above.
Fig. 2 is a gel electrophoregram according to Embodiment 1 of the present disclosure.
Fig. 3 is a gel electrophoregram according to Embodiment 2 of the present disclosure.
Fig. 4 is a UPLC chromatogram according to Embodiment 2 of the present disclosure.
Fig. 5 is a UPLC chromatogram according to Embodiment 2 of the present disclosure.
Fig. 6 is a UPLC chromatogram according to Embodiment 3 of the present disclosure.
Fig. 7 is a mass spectrogram according to Embodiment 3 of the present disclosure.
Fig. 8 is a UPLC chromatogram according to Embodiment 4 of the present disclosure.
Fig. 9 is a mass spectrogram according to Embodiment 4 of the present disclosure.
Fig. 10 is a UPLC chromatogram according to Embodiment 5 of the present disclosure.
Fig. 11 is a gel electrophoregram according to Embodiment 6 of the present disclosure.

### Detailed Description of the Embodiments

It is to be noted that the embodiments in the present application and the features in the embodiments may be combined with one another without conflict. The present disclosure will be described below in detail with reference to the embodiments.

As mentioned in the Background, there are no studies on the preparation of RNA containing a phosphorothioate bond using biological methods such as enzyme catalysis, etc. in the prior art. For an RNA ligase, different RNA ligases have different requirements for the lengths, modifications, single/double-stranded states, etc. of substrates for ligation, and in particular, when difficult substrate ligation containing phosphorothioate or non-natural nucleotides is catalyzed, there are problems of low catalytic activity, incorrect product ligation, etc. The inventor in the present application attempts to develop a method for preparing RNA containing a phosphorothioate bond using an RNA ligase, thereby proposing a series of protective solutions of the present application.

In a first typical implementation of the present application, a method for preparing an RNA containing a phosphorothioate bond is provided. The preparation method includes: the ligation of 3' hydroxyl of a substrate with 5' phosphorothioate is catalyzed with an RNA ligase, so as to form the RNA containing the phosphorothioate bond, where the 5' phosphorothioate includes 5' phosphate containing a thio modification, and the thio modification includes the replacement of an oxygen atom in P-OH or P=O of the 5' phosphate by a sulfur atom, thereby forming P-SH or P=S accordingly; and the RNA ligase includes any one or more enzymes of RNA ligase family 1, 2 or 3. The RNA ligase includes one or more of RNA ligases shown in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21 or SEQ ID NO: 22; or an enzyme having more than 70%, preferably, more than 75%, more than 80%, more than 85%, more than 90%, more than 95%, more than 98%, more than 99%, more than 99.5%, or more than 99.9% identity to any one of the RNA ligases shown in SEQ ID NO: 1 to SEQ ID NO: 22, and having a protein that catalyzes the activity of the ligation of the 3' hydroxyl and the 5' phosphorothioate.

The ligation of the 3' hydroxyl and 5' phosphorothioate can be catalyzed with the above RNA ligase, so as to form the phosphorothioate bond similar to a phosphodiester bond, thereby obtaining the RNA containing a phosphorothioate bond through preparation. In the present application, 5' (5' terminal) phosphoric acid/phosphate group/phosphate refers to a phosphate group on a C atom at position 5 of a 5' terminal nucleotide of a nucleic acid chain. A 5' phosphorothioate modification includes a 5' thio modification, which includes the replacement of an oxygen atom in P-OH or P=O of the 5' phosphate by a sulfur atom, thereby forming P-SH or P=S accordingly, and does not specifically refers to P-SH or P=S. The structure of the 5' phosphorothioate modification includes one or more of 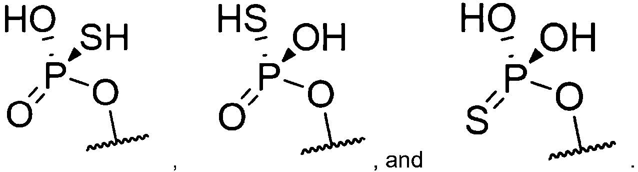
SEQ ID NO: 1(ligase-1, *Escherichia phage* T4, RNA ligase 1(RNA ligase family 1, Rnl1)): 
MQELFNNLMELCKDSQRKFFYSDDVSASGRTYRIFSYNYASYSDWLLPDALECRGIMFEMDG
EKPVRIASRPMEKFFNLNENPFTMNIDLNDVDYILTKEDGSLVSTYLDGDEILFKSKGSIKSEQALMA
NGILMNINHHRLRDRLKELAEDGFTANFEFVAPTNRIVLAYQEMKIILLNVRENETGEYISYDDIYKDA
TLRPYLVERYEIDSPKWIEEAKNAENIEGYVAVMKDGSHFKIKSDWYVSLHSTKSSLDNPEKLFKTII
DGASDDLKAMYADDEYSYRKIEAFETTYLKYLDRALFLVLDCHNKHCGKDRKTYAMEAQGVAKGA
GMDHLFGIIMSLYQGYDSQEKVMCEIEQNFLKNYKKFIPEGY.
SEQ ID NO: 2(ligase-2, *Salmonella phage* STP4-a, RNA ligase 2(RNA ligase family 2, Rnl2)): 
MFKKYSSLENHYNNKFISKIRFEGKDGGLWVAREKIHGTNFSIIVSKDSVSACKRSGPILPSES
FYGHEIILKNYDESIKTIQRCMNANELGSVSSYQIFGEFAGQGIQKEVDYGEKDFYVFDILVNTQNG
NVLYMDDMMMTSFCNEFGFKMAPFIGCGSFDELIQLPNNFTSVIKAYNEAAKDDLKEVNLCVFDPL
VTDDNVAEGYVLKPVYPDFFNNGTRIAIKSKNSRFTEKKKSDKPIKPKAVLTSNDSTVLANLCEYST
WNRVSNVISHIGEVKAKDFGKVMGLTIQDIFIEAKREGVDIVHADNPDLVKRELQTVVSNTIREKWL
 EIVS.
SEQ ID NO: 3(ligase-3, *Escherichia phage* T4, RNA ligase 2): 
MFKKYSSLENHYNSKFIEKLYSLGLTGGEWVAREKIHGTNFSLIIERDKVTCAKRTGPILPAEDF
FGYEIILKNYADSIKAVQDIMETSAVVSYQVFGEFAGPGIQKNVDYCDKDFYVFDIIVTTESGDVTYV
DDYMMESFCNTFKFKMAPLLGRGKFEELIKLPNDLDSVVQDYNFTVDHAGLVDANKCVWNAEAK
GEVFTAEGYVLKPCYPSWLRNGNRVAIKCKNSKFSEKKKSDKPIKAKVELSEADNKLVGILACYVTL
NRVNNVISKIGEIGPKDFGKVMGLTVQDILEETSREGITLTQADNPSLIKKELVKMVQDVLRPAWIEL
VS.
SEQ ID NO: 4(ligase-4, *Vibrio phage* KVP40, RNA ligase 2): 
MSFVKYTSLENSYRQAFVDKCDMLGVREWVALEKIHGANFSFIVEFKPNEAQDGAEFTVTPA
KRTSTIGANVMGDYDFYGCTSVVEAHTAKMEAISNWLWARGIINVGETIIVYGELAGKGVQKEVNY
GDKDFWAYDILLPETGKFLDWDVVLEACEFAKVKTTHEIARGTLDELLRIDPLFRSFHTPADVDGDN
VAEGFVVKQLRNEKRLHNGSRAILKVKNDKFKEKKNKAGKTPRAAVVLTEEQEKLHAAFSCYLTEN
RLRNVLSKLETVTQKQFGMISGLFIKDAKDEFERDELNETAIARDDWDVIKRSLTNIANEILRKNWLD
ILDGNF.
SEQ ID NO: 5(ligase-5, *Yersinia phage* fPS-2, RNA ligase 2): 
MFKKYSSLENHYNSKFIEKLYSLGLTGGEWVAREKIHGTNFSLIIERDKVTCAKRTGPILPAEDF
FGYEIILKNYADSIKAVQDIMETSAVVSYQVFGEFAGPGIQKNVDYGDKDFYVFDIIVTTESGGVTYV
DDYMMESFCNSFGFKIAPLLGRGKFEDLIKLPNDLDSVVQDYNFTVDHAGLVDANKCVWNAEAKG
EVFTAEGYVLKPCYPSWLPNRNRVAIKCKNSKFSEKKKSDKPIKAKVELSEADNKLVGILACYVTLN
RVNNVISKIGEIGPKDFGKVMGLTVQDILEETSREGITLTQADNPSLIKKELVKMVQDVLRPAWIELV
S.
SEQ ID NO: 6(ligase-6, *Vibrio phage* nt-1, RNA ligase 2): 
MSFVKYTSLENSYRQAFVDKCDMLGVRDWVALEKIHGANFSFIVEFDGGYTVTPAKRTSIIGA
TATGDYDFYGCTSVVEAHKEKVELVANFLWLNEYINLYEPIIIYGELAGKGIQKEVNYGDKDFWAFDI
FLPQREEFVDWDTCVAAFTNAEIKYTKELARGTLDELLRIDPLFKSLHTPAEHEGDNVAEGFVVKQL
HSEKRLQSGSRAILKVKNEKFKEKKKKEGKTPTKLVLTPEQEKLHAEFSCYLTENRLKNVLSKLGTV
NQKQFGMISGLFVKDAKDEFERDELNEVAIDRDDWNAIRRSLTNIANEILRKNWLNILDGNF.
SEQ ID NO: 7(ligase-7, *Shigella phage* Sf22, RNA ligase 1): 
MQELFNNLMELCKDSQRKFFYSDDVSASGRTYRIFSYNYASYSDWLLPDALECRGIMFEMDG
EKPVRIASRPMEKFFNLNENPFTMNIDLNDVDYILTKEDGSLVSTYLDGDEILFKSKGSIKSEQALMA
NGILMNINHHQLRDRLKELAEDGFTANFEFVAPTNRIVLAYQEMKIILLNIRENETGEYISYDDIYKDA
ALRPYLVERFEVDSPKWIEEAKNAENIEGYVAVMKDGSHFKIKSDWYVSLHSTKSSLDNPEKLFKTI
IDGASDDLKAMYADDEYSYRKIEAFETTYLKYLDRALFLVLDCHNKHCGKDRKTYAMEAQGVAKGA
 GMEHLFGIIMSLYQGYDSQEKVMCEIEQNFLKNYKKFIPEGY.
SEQ ID NO: 8(ligase-8, *Vibrio phage* VH7D, RNA ligase 1): 
MNVQELYKNLMSLADDAEGKFFFADHLSPLGEKFRVFSYHIASYSDWLLPGALEARGIMFQLD
DNDEMIRIVSRPMEKFFNLNENPFTMELDLTTTVQLMDKADGSLISTYLSGENFALKSKTSIFSEQA
VAANRYIKKPENRDLWEFCDDCTQAGLTVNMEWCAPNNRIVLEYPEAKLVILNIRDNETGDYVSFD
DIPQSALMRVKQWLVDEYDPATAHEPDFVEKLRDTKGIEGMILRLANGQSVKIKTQWYVDLHSQKD
SVNVPKKLVTTILNGNHDDLYALFADDKPTIERIREFDSHVTKTLTNSFNAVRQFYARNRHLARKDYA
IAGQKVLKPWEFGVAMIAYQKQTVEGVYESLVTAYLKRPELAIPEKYLNGV.
SEQ ID NO: 9(ligase-9, *Bacteriophage* dhaeg, RNA ligase 2): 
MFKKYSSLENHYNSKFIEKLRTNGLTGGEWVAREKIHGTNFSLIIERDAVTCAKRTGPILPAEDF
YGYEIVLKNYADSIKSIQDIMETSAAVSYQVFGEFAGTGIQKNVDYGDKDFYVFDIIVTTESGDVTYV
DDYMMESFCKTFKFKMAPLLGRGKFEDLIKLPNDLDSVLPDYNFTVDNVGLAEANAHVWNAEAK
GEVFTAEGYVLKPCYPLWLPNGNRVAIKCKNSKFSEKKKTDKPIKVAVVLSQDDLDLLQQFTDYVT
VNRINNVISKIGEVSPKDFGKVMGLTVQDILEEAAREELELTDAENPVEVKKQLIECVKDTLRAVWIE
LVSK.
SEQ ID NO: 10(ligase-10, *Klebsiella phage* KP27, RNA ligase 2): 
MFKKYSSLTNHYEGKFINGVIMNGLTGGVWVAREKIHGANFSFITDDGITVTPAKRTDVVKPAE
DFYGCSAVVAKYSPGIRKMWETLKKTGTYDDLVIQVYGEFAGRGVQKDVDYGEKDFYVFDIRVNG
EFLPDNLCSLISRSHGLKMAPLLGYGTFEEIKELPITFESVVNKANSGIGSDNTVYGEFVYPIMDVEE
GNIAEGFVMKPVSPAFMPNGERVAIKCKTTKFTEKKAKKATRFNAPVSLSEKDKNQLDEFVCYLTE
NRVKNVLSKLDLASITAKDFGRIMGLTVQDAIEEISRNHGPFLEQFEDPAMAKKLFVTEAQNMIRPV
WGKILNHEF.
SEQ ID NO: 11(ligase-11, *Vibrio phage* V05, RNA ligase 1): 
MTTQELYNHLMTLTDDAEGKFFFADHISPLGEKLRVFSYHIASYSDWLLPGALEARGIMFQLD
EQDKMVRIVSRPMEKFFNLNENPFTMDLDLTTTVQLMDKADGSLISTYLTGENFALKSKTSIFSEQA
VAANRYIKLPENRDLWEFCDDLTQAGCTVNMEWCAPNNRIVLEYPEAKLVILNIRDNETGDYVSFD
DIPLPALMRVKKWLVDEYDPETAHADDFVEKLRATKGIEGMILRLANGQSVKIKTQWYVDLHSQKD
SVNVPKKLVTTILNNNHDDLYALFADDKPTIDRIREFDSHVSKTVSASFHAVSQFYVKNRHMSRKDY
AIAGQKTLKPWEFGVAMIAYQNQTVEGVYEALVGAYLKRPELLIPEKYLNEA.
SEQ ID NO: 12(ligase-12, *Rhodothermus phage* RM378, RNA ligase 1): 
MESMNVKYPVEYLIEHLNSFESPEVAVESLRKEGIMCKNRGDLYMFKYHLGCKFDKIYHLACR
GAILRKTDSGWKVLSYPFDKFFNWGEELQPEIVNYYQTLRYASPLNEKRKAGFMFKLPMKLVEKL
DGTCVVLYYDEGWKIHTLGSIDANGSIVKNGMVTTHMDKTYRELFWETFEKKYPPYLLYHLNSSYC
YIFEMVHPDARVVVPYEEPNIILIGVRSVDPEKGYFEVGPSEEAVRIFNESGGKINLKLPAVLSQEQN
 
YTLFRANRLQELFEEVTPLFKSLRDGYEVVYEGFVAVQEIAPRVYYRTKIKHPVYLELHRIKTTITPE
KLADLFLENKLDDFVLTPDEQETVMKLKEIYTDMRNQLESSFDTIYKEISEQVSPEENPGEFRKRFA
LRLMDYHDKSWFFARLDGDEEKMQKSEKKLLTERIEKGLFK.
SEQ ID NO: 13(ligase-13, *Citrobacter phage* CkP1, RNA ligase 1): 
MKKLFKNLMALCDEADESKFFYRDDISPSGLKYRIFSYNYASYSDWIRPDALECRGIMFEMID
DKPVRIASRPMEKFFNLNETPFTMNLDLSKAKYMLDKADGSLVSTFLDGNILKFKSKSSIKSEQSYF
STAMLTESRHEALLARLLDLASDGFTANFEYVSPDNRIVLPYQEKQLILLNIRDNDTGEYVDYEDIFK
DGVLRQYLVQRHEITDSNFVEDIRKLTDIEGFVFVMEDGLRFKLKTDWYCALHHTKDSITNNERLFE
SIVNNASDDLKAMFAGDEFAVNKINKFEENYLKYLGESLHLINTTYHELRGRDRKDYAIESQNRANK
SGLGFLFSIIMKMYNGGMDHDTRVKNLSELFMKNYKQFVPKEFI.
SEQ ID NO: 14(ligase-14, *Escherichia phage* JN02, RNA ligase 1): 
MEKLYYNLLSLCKSSSDRKFFYSDDVSPIGKKYRIFSYNFASYSDWLLPDALECRGIMFEMDG
ETPVRIASRPMEKFFNLNENPFTLSINLDDVKYLMTKEDGSLVSTYLDGGTVRFKSKGSIKSDQAVS
ATSILLDIDHKNLADRLLELCNDGFTANFEYVAPTNKIVLTYPEKRLILLNIRDNNTGEYIEYDDIYLDP
VFRKYLVDRFEVPEGDWTSDVKSSTNIEGYVAVMKDGSHFKLKTDWYVALHTTRDSISSPEKLFLA
IVNGASDDLKAMYADDEFSFKKVELFEKAYLDFLDRSFYICLDTYDKHGKDRKTYAIEAQAVCKGA
QTPWLFGIIMNLYQGGSKEQMMTALESVFIKNHKNFIPEGY.
SEQ ID NO: 15(ligase-15, *Klebsiella phage* KP179, RNA ligase 1): 
MLELYKNLMNLCESSEVAKFFYKDFTGPMDGKFRVFSYHYASYSEWLKPDALECRGIMFEMD
GDTPIRIASRPMEKFFNLNENPLTMGIDISDVEYIMDKADGSLVSSYVDDGYLYLKSKTSLYSDQAR
QASALLNSEEYSSLHQVILELTLDGYTVNMEFVSPNNRVVLAYQEPQLFVLNVRNNTTGEYIKYDD
LYANAKIRPYLINAYGISDPTTWVEGVRELEGVEGYIAVLNTGQRFKVKTEWYSALHHTKDSITSNE
RLFASVVSANSDDLRSLFAGDEYAIKKISAFEQAYLDYLGKSLELCQSFYDEYRGRARKDYAIAAQK
ATVNQRHLFGVIMNMYEGTVDVDKLLKDLERVFLKYWAGYVPKEYEKEIELSEE.
SEQ ID NO: 16(ligase-16, *Vibrio phage* nt-1, RNA ligase 1): 
MNELYNNLMTLAESAEGKFFFADHLSPQGEKFRVFSYHIASYSDWLLPDALEARGIMFQLDEN
DEMIRIVSRPMEKFFNLGENPFTMDLDLTTTVQLMDKADGSLISTYLTGDNFALKSKTSIYSEQAVA
ANRYIKHVDNRDLWEFCDDCAAQNFTVNMEWCAPNNRIVLEYTEPKLIILNIRDNETGQYVSFDDIP
IGALTRIKKWLVDEYDPMTAHVDDFVETLKAKKGIEGMILRLASGQSVKIKTTWYVDLHAQKDSVNS
PKKLVTTILNKNHDDLYALFADDKPTIERIREFDDHVSKKVVESFHAVSQYYTKNRHLSRKDFAIAGQ
KALKPWEFGVAMIAYQNQTVEGVVTMLINAYLKRPELLIPEKYLSEV.
SEQ ID NO: 17(ligase-17, *Vibrio phage* VH7D, RNA ligase 2): 
MSFVKYTSLENSYRQAFVDKCDMLGVKEWVALEKIHGANFSFIVEFKPSTEEVPGEMSVTPA
KRTSTIGANAMGDYDFYGCTSVVEAHIEKMQDISNWLFANDFIKNDETIIVYGELAGKGIQKEVNYG
 
DKDFWAYDILCPETGEFLDWDVVLKACKFAGVKTTHEIARGTLDELLKIDPLFRSFHTPADVDSENV
AEGFVVKQLKAEKRLHNGSRAILKVKNEKFKEKKNKQGKTPRAKVVLTEEQEKLHAAFSCYLTENR
LRNVLSKIGKVEAKQFGMVSGLFVKDAKDEFERDERDEVAIPRDDWDVIKRSLVNVANEILRKNWL
NIVDGTF.
SEQ ID NO: 18(ligase-18, *Klebsiella phage* KP15, RNA ligase 2): 
MFKKYSSLTNHYEGKFINGVIMNGLTGGVWVAREKIHGANFSFITDDGITVTPAKRTDVVKPAE
DFYGCSAVVAKYSPGIRKMWETLKKTGTYDDLVIQVYGEFAGRGVQKDVDYGEKDFYVFDIRVNG
EFLPDNLCSLISRSHGLKMAPLLGYGTFEEIKELPITFESVVNKANSGIGSDNTVYGEFVYPIMDVEE
GNIAEGFVMKPVSPAFMPNGERVAIKCKTTKFTEKKAKKATRFNAPVSLSEKDKNQLDEFVCYLTE
NRVKNVLSKLDLASITAKDFGRIMGLTVQDAIEEISRNHGPFIEQFEDPAMAKKLFVTEAQNMIRPV
WGKILNHEF.
SEQ ID NO: 19(ligase-19, *Vibrio phage* JN02, RNA ligase 2): 
MFKKYSSLENHYNSKFIEKLYTNGLTTGVWVAREKIHGTNFSLIIERDNVTCAKRTGPILPAEDF
YGYEIVLKKYDKAIKAVQEVMESISTSVPVSYQVFGEFAGGGIQKGVDYGEKDFYVFDIIINTESDDT
YYMSDYEMQDFCNTFGFKMAPMLGRGTFDSLIMIPNDLDSVLAAYNSTASEDLVEANNCVFDANVI
GDNTAEGYVLKPCFPKWLSNGTRVAIKCKNSKFSEKKKSDKPVKTQVPLTEIDKNLLDVLACYVTL
NRVNNVISKIGTVTPKDFGKVMGLTVQDILEETSREGIVLTSSDNPNLVKKELVRMVQDVLRPAWIE
LVS.
SEQ ID NO: 20(ligase-20, *Escherichia phage* JS98, RNA ligase 2): 
MFKKYSSLENHYNSKFIEKLRTNGLTGGEWVAREKIHGTNFSLIIERDAVTCAKRTGPILPAEDF
YGYEIVLKNYADSIKSVQHLIESINYQSYQIYGELAGPGIQKNVDYGDKDFYVFDIRVTKEDGTESVL
TDTLMEAFCIIHKFKVAPCLATGSFEDLIKLPNDFDSVIPDYNFAVDNAGLTIANSTDFIPKVEGKVFT
AEGFVLKPDIPTWLPNGNRVAIKCKNSKFSEKKKSDKPIKAAVVLSQDDMDLMWQFTDYVTVNRIN
NVISKIGEVSKKDFGKVMGLTVQDILEEAAREELELTDAENPVEVKKQLVECVKDTLRAVWIELVS.
SEQ ID NO: 21(ligase-21, *Escherichia phage* JS98, RNA ligase 3(RNA ligase family 3, Rnl3)): 
MVSSVYKEILVKLGLTEDRIETLEMKGGIIEDEFDGIRYVRFKDSAGKLRRGTVVIDEEYVIPGF
PHIKRIINLRSGIRRIFKRGEFYVEEKVDGYNVRVVMYKGKMLGITRGGFICPFTTERIPDFVPQEFF
KDNPNLILVGEMAGPESPYLVEGPPYVKEDIQFFLFDVQEIKTGRSLPVEERLKIAEEYGINHVEVF
GKYTKDDVDELYQLIERLSKEGREGIIMKSPDMKKIVKYVTPYANINDIKIGARVFYELPPGYFTSRIS
RLAFYLAEKRIKGEEFERVAKELGSALLQPFVESIFDVEQEEDIHELFKVRVKRIETAYKMVTHFEKL
GLKIEIVDIEEIKDGWRITFKRLYPDATNEIRELIGGKAFVD.
SEQ ID NO: 22(ligase-22, *fungus Deinococcus radiodurans*, RNA ligase 2):

MAERQVIKERAQLFPHPNAERLELCKVGTFQLVVRKGEYRDGDPIVIAPEKAVLPPQLAGLYT
NADTGASYLHGAEKNRVGSVRLRGEVSQGVILPLDGLEDAPFGEDLAERLGITFWEPPVPVSMAG

EVEPRPPAQHYKHHDVEQFGIYVSEFASGEEVMVTEKLHGTQGVYFRTAEGRWLVTSKGLSRGG
LTLREAASNVYWQAARNSNLFAEADAAFSGGEVQIFGEVVPVQKGFSYGQHKPTVFVFKVVYDGL
RLPRRDWPQWVLDHAVPVLYEGPFDEATVRKLRGGLETVSGKGLHIREGVVVAPKVPRFAADGS
DLSVKLISDAYAKKETGEEYS.

Identity in the specification refers to "identity" between amino acid sequences or nucleotide sequences, that is, the total of ratios of the amino acid residues or nucleotides of the same type in the amino acid sequences or nucleotide sequences. The identity of the amino acid sequences or nucleotide sequences may be determined with comparison programs such as a Basic Local Alignment Search Tool (BLAST), FASTA, etc.

Active sites, active pockets, active mechanisms, protein structures, etc. of proteins having more than 70%, 75%, 80%, 85%, 90%, 95%, 99% (e.g., more than 85%, 86%, 87%, 88%, 89%, 90%, 91%, 92%, 93%, 94%, 95%, 96%, 97%, 98%, 98.5%, 99%, 99.5%, 99.6%, 99.7%, 99.8%, or even more than 99.9%) identity and having the same functions are probably the same as the protein provided the sequence in a).

As used herein, the amino acid residues are abbreviated below: Alanine (Ala; A), Asparagine (Asn; N), Aspartic acid (Asp; D), Arginine (Arg; R), Cysteine (Cys; C), Glutamate (Glu; E), Glutamine (Gln; Q), Glycine (Gly; G), Histidine (His; H), Isoleucine (Ile; I), Leucine (Leu; L), Lysine (Lys; K), Methionine (Met; M), Phenylalanine (Phe; F), Proline (Pro; P), Serine (Ser; S), Threonine (Thr; T), Tryptophan (Trp; W), Tyrosine (Tyr; Y), and Valine (Val; V).

For rules of substitution, replacement, etc., if amino acids are similar in nature, replacements have similar effects. For example, in the homologous protein, conservative amino acid replacements may occur. "Conservative amino acid replacement" includes, but is not limited to:
Hydrophobic amino acids (Ala, Cys, Gly, Pro, Met, Val, Ile, and Leu) are substituted by other hydrophobic amino acids.

Hydrophobic amino acids with bulky side chains (Phe, Tyr, and Trp) are substituted by other hydrophobic amino acids with bulky side chains.

Amino acids with positively charged side chains (Arg, His, and Lys) are substituted by other amino acids with positively charged side chains.

Amino acids with polarized and uncharged side chains (Ser, Thr, Asn, and Gln) are substituted by other amino acids with polarized and uncharged side chains.

Person skilled in the art may also make conservative substitutions of the amino acids according to amino acid substitution rules known to person skilled in the art, such as a "blosum62 scoring matrix" in the prior art.

In a preferred embodiment, the substrate includes a single-stranded RNA or a double-stranded RNA, and correspondingly, the RNA containing the phosphorothioate bond includes a single-stranded RNA containing a phosphorothioate bond or a double-stranded RNA containing a phosphorothioate bond; and one, two or more kinds of substrates are provided.

The length of the above substrate is ≥2 nt, including, but not limited to, 2, 5, 10, 15, 20, 30, 40, 50, 70, 100, 150, 200, 300, 500, 1000, 2000, and 5000 nt. Ligation may still be performed when the length of a substrate increases, though this reduces connection efficiency to some extent.

One or two substrates are used in the above preparation method. When there is one substrate, the 3' terminal of the substrate is ligated to hydroxyl, and the 5' terminal is ligated to phosphorothioate. The 3'-hydroxyl group of the substrate can be ligated to the 5' phosphorothioate with the above preparation method, causing the self-cyclization of the substrate, thereby forming the RNA containing the phosphorothioate bond. When there are two substrates, the substrates include a first substrate and a second substrate, the 3' terminal of the first substrate is ligated to hydroxyl, and the 5' terminal of the second substrate is ligated to phosphorothioate. The 3' terminal of the first substrate can be ligated to the 5' terminal of the second substrate via a phosphorothioate bond with the above preparation method, thereby forming the RNA containing the phosphorothioate bond.

In a preferred embodiment, when one substrate is provided and the substrate is the single-stranded RNA, the 5' terminal of the substrate is the 5' phosphorothioate, and the 3' terminal of the substrate is the 3' hydroxyl, and the 5' terminal of the substrate and the 3' terminal of the substrate are ligated and cyclized under the catalysis of the RNA ligase, so as to obtain a cyclic single-stranded RNA containing a phosphorothioate bond. Preferably, the cyclization of the single-stranded RNA is guided using a splint, the splint specifically binds to at least three bases from the 3' terminal to the 5' terminal of the single-stranded RNA, and the splint specifically binds to at least three bases from the 5' terminal to the 3' terminal of the single-stranded RNA, and bases of the splint which specifically bind to the 3' terminal of the single-stranded RNA are adjacent to bases which specifically bind to the 5' terminal of the single-stranded RNA.

The 5' terminal and 3' terminal of the substrate can be ligated end to end with the above preparation method, so as to form the single-stranded cyclic RNA. The preparation of the above single-stranded cyclic RNA also includes the use of a splint to guide the cyclization of the single-stranded RNA. The splint can specifically bind to bases of the 5' terminal and 3' terminal of the substrate single-stranded RNA, respectively, such that a space distance between the 5' terminal and the 3' terminal is shortened with the splint, thereby achieving the catalytic ligation of the RNA ligase.

The above RNA ligase includes, but is not limited to, any one or more of ligase-1(SEQ ID NO: 1), ligase-4(SEQ ID NO: 4), ligase-7(SEQ ID NO: 7), ligase-8(SEQ ID NO: 8), ligase-9(SEQ ID NO: 9), ligase-10(SEQ ID NO: 10), ligase-11(SEQ ID NO: 11), ligase-12(SEQ ID NO: 12), ligase-13(SEQ ID NO: 13), ligase-14(SEQ ID NO: 14), ligase-15(SEQ ID NO: 15), ligase-16(SEQ ID NO: 16), ligase-18(SEQ ID NO: 18), ligase-19(SEQ ID NO: 19).

In a preferred embodiment, when two kinds of substrates are provided and the substrates are the single-stranded RNA, the substrates include a first substrate and a second substrate, the 3' terminal of the first substrate is the 3' hydroxyl, and the 5' terminal of the second substrate is the 5' phosphorothioate; and the 3' terminal of the first substrate and the 5' terminal of the second substrate are ligated under the catalysis of the RNA ligase to obtain a linear single-stranded RNA containing a phosphorothioate bond. Preferably, the 3' terminal of the second substrate is a 3' terminal protecting group, and the 5' terminal of the first substrate is a 5' terminal protecting group.

With the above preparation method, the first substrate can be ligated to the second substrate, so as to form the single-stranded linear RNA. The above 5' protecting group includes, but is not limited to, groups such as hydroxyl, an N-acetyl galactosamine group, -O-NH₂, an azido group, a cyanoethyl group, an allyl group or a 2-nitrobenzyl group, etc. The above 3' protecting group includes, but is not limited to, groups such as a phosphate group, an N-acetyl galactosamine group, -O-NH₂, an azido group, a cyanoethyl group, an allyl group or a 2-nitrobenzyl group, etc. In order to prevent the self-cyclization of the substrate, a single substrate cannot have the 5' phosphorothioate and the 3' terminal hydroxyl at the same time, that is, the above 3' protecting group may not be the hydroxyl, and the above 5' protecting group may not be phosphate.

The above RNA ligase includes, but is not limited to, any one or more of ligase-1(SEQ ID NO: 1), ligase-2(SEQ ID NO: 2), ligase-3(SEQ ID NO: 3), ligase-4(SEQ ID NO: 4), ligase-5(SEQ ID NO: 5), ligase-6(SEQ ID NO: 6), ligase-7(SEQ ID NO: 7), ligase-8(SEQ ID NO: 8), ligase-9(SEQ ID NO: 9), ligase-10(SEQ ID NO: 10), ligase-11(SEQ ID NO: 11), ligase-12(SEQ ID NO: 12), ligase-13(SEQ ID NO: 13), ligase-14(SEQ ID NO: 14), ligase-15(SEQ ID NO: 15), ligase-16(SEQ ID NO: 16), ligase-17(SEQ ID NO: 17), ligase-18(SEQ ID NO: 18), ligase-19(SEQ ID NO: 19), and ligase-20(SEQ ID NO: 20).

In a preferred embodiment, when two kinds of substrates are provided and the substrates are the double-stranded RNA, each substrate includes a sense strand and an antisense strand, and the substrates include a first substrate and a second substrate; the 3' terminal of the sense strand of the first substrate is the 3' hydroxyl, and the 5' terminal of the antisense strand of the first substrate is the 5' phosphate; the 5' terminal of the sense strand of the second substrate is the 5' phosphate, and the 3' terminal of the antisense strand of the second substrate is the 3' hydroxyl; at least one of the 5' phosphate of the antisense strand of the first substrate and the sense strand of the second substrate is the 5' phosphorothioate; the 3' terminal of the sense strand of the first substrate and the 5' terminal of the sense strand of the second substrate are ligated under the catalysis of the RNA ligase; and the 5' terminal of the antisense strand of the first substrate and the 3' terminal of the antisense strand of the second substrate are ligated under the catalysis of the RNA ligase, so as to obtain linear double-stranded RNA containing a phosphorothioate bond. Preferably, the 3' terminal of the antisense strand of the first substrate and the 3' terminal of the sense strand of the second substrate are both 3' terminal protecting groups. Preferably, the 5' terminal of the sense strand of the first substrate and the 5' terminal of the antisense strand of the second substrate are both 5' protecting groups. Preferably, the first substrate and the second substrate are both double-stranded RNA with the lengths of a sense strand and antisense strand being different, the 3' terminal of the sense strand of the first substrate is complementarily paired with the 3' terminal of the antisense strand of the second substrate, or the 5' terminal of the antisense strand of the first substrate is complementarily paired with the 5' terminal of the sense strand of the second substrate. Thereby, the 3' terminal of the sense strand of the first substrate is adjacent to the 5' terminal of the sense strand of the second substrate, and the 5' terminal of the antisense strand of the first substrate is adjacent to the 3' terminal of the antisense strand of the second substrate.

With the above preparation method, two pieces of double-stranded RNA can be ligated through two phosphodiester bonds, and at least one of the phosphodiester bonds is a phosphorothioate bond, thereby obtaining the linear double-stranded RNA. Preferably, in order to prevent the self-cyclization of the substrate, the corresponding 3' terminal on the single strand containing 5' phosphate is not the hydroxyl. When the above first substrate and second substrate both are the double-stranded RNA with the lengths of the sense strand and antisense strand being different and partial base in the first substrate can be complementarily paired with partial base in the second substrate, it indicates that cohesive ends that can be complementarily paired with each other are present in the first substrate and second substrate. Good ligation efficiency can be achieved with such substrates for ligation.

The above RNA ligase includes, but is not limited to, any one or more of ligase-2(SEQ ID NO: 2), ligase-3(SEQ ID NO: 3), ligase-4(SEQ ID NO: 4), ligase-5(SEQ ID NO: 5), ligase-6(SEQ ID NO: 6), ligase-9(SEQ ID NO: 9), ligase-10(SEQ ID NO: 10), ligase-11(SEQ ID NO: 11), ligase-14(SEQ ID NO: 14), ligase-17(SEQ ID NO: 17), ligase-18(SEQ ID NO: 18), ligase-19(SEQ ID NO: 19), ligase-20(SEQ ID NO: 20), ligase-22(SEQ ID NO: 22).

The above 5' protecting group includes, but is not limited to, groups such as hydroxyl, an N-acetyl galactosamine group, a phosphate group, -O-NH₂, an azido group, a cyanoethyl group, an allyl group or a 2-nitrobenzyl group, etc. The above 3' protecting group includes, but is not limited to, groups such as a phosphate group, an N-acetyl galactosamine group, -O-NH₂, an azido group, a cyanoethyl group, an allyl group or a 2-nitrobenzyl group, etc. The 3' terminal of the antisense strand of the first substrate and the 3' terminal of the sense strand of the second substrate are each independently selected from the above 3' protecting groups, which are not the hydroxyl. The 5' terminal of the sense strand of the first substrate and the 5' terminal of the antisense strand of the second substrate are each independently selected from the above 5' protecting groups, which are not the phosphate.

In a preferred embodiment, the preparation method further includes: a splint is used to guide the ligation between the first substrate and the second substrate. Preferably, the splint specifically binds to at least three bases from the 3' terminal to the 5' terminal of the first substrate, and the splint specifically binds to at least three bases from the 5' terminal to the 3' terminal of the second substrate. Preferably, bases of the splint specifically binding to the 3' terminal of the first substrate are adjacent to bases specifically binding to the 5' terminal of the second substrate.

In the above preparation method, the splint can be used to guide the ligation between the first substrate and the second substrate. The first substrate and the second substrate both can specifically bind to the splint, a nick exists between the 3' terminal of the first substrate and the 5' terminal of the second substrate after base-pairing, and the above RNA ligase can ligate the nick using the phosphorothioate bond, thereby realizing the preparation of the RNA containing the phosphorothioate bond. The above splint includes a DNA sequence or an RNA sequence.

The above RNA ligase includes, but is not limited to, any one or more of ligase-2(SEQ ID NO: 2), ligase-3(SEQ ID NO: 3), ligase-4(SEQ ID NO: 4), ligase-5(SEQ ID NO: 5), ligase-6(SEQ ID NO: 6), ligase-7(SEQ ID NO: 7), ligase-8(SEQ ID NO: 8), ligase-9(SEQ ID NO: 9), ligase-10(SEQ ID NO: 10), ligase-11(SEQ ID NO: 11), ligase-14(SEQ ID NO: 14), ligase-16(SEQ ID NO: 16), ligase-17(SEQ ID NO: 17), ligase-18(SEQ ID NO: 18), ligase-19(SEQ ID NO: 19), ligase-20(SEQ ID NO: 20), ligase-21(SEQ ID NO: 21), and ligase-22(SEQ ID NO: 22).

In a preferred embodiment, the preparation method can be repeatedly used to ligase three or more substrates for a plurality of times, the number of the substrates ligated each time is two, and the substrates are sequentially ligated to obtain the RNA containing a phosphorothioate bond.

In a preferred embodiment, the preparation method includes: a splint is used to guide the ligation of three or more substrates. Preferably, the splint specifically binds to at least three bases of each of the substrates. Preferably, bases of the substrates specifically binding to the splint are adjacent to each other, and the adjacent bases are located at the 5' terminal and 3' terminal of different substrates, respectively; the 5' terminal is the 5' phosphate, and at least one of the 5' phosphate is the 5 phosphorothioate, more preferably, all the 5' phosphate is the 5 phosphorothioate; and all the 3' terminal is the hydroxyl.

With the above preparation method, the plurality of substrates may be ligated at the same time with one splint. The above RNA ligase can ligate the nicks among the plurality of substrates, thereby obtaining the RNA containing one or more phosphorothioate bonds.

Fig. 1 is a schematic diagram of the above preparation method, where a in Fig. 1 is a schematic diagram of the ligation of two pieces of single-stranded RNA using the above method; b in Fig. 1 is a schematic diagram of the use of a splint to guide the ligation of two single-stranded RNA substrates using the above method; c in Fig. 1 is a schematic diagram of two pieces of double-stranded RNA using the above method; and d in Fig. 1 is a schematic diagram of formation of single-stranded cyclic RNA with the above method to cyclize the substrate. As shown in d in Fig. 1, the cyclization of the single-stranded RNA includes using the splint to guide the cyclization of the single-stranded cyclic RNA, and not using the splint, but using the RNA ligase to directly catalyze the cyclization of the single-stranded RNA.

In a preferred embodiment, a catalytic system of the RNA ligase includes 1-20000 µM of the substrate, 1-40000 µM of ATP, 5-100 mM of Tris-HCl, 0.1-20 mM of bivalent metal ions, 0-10 mM of dithiothreitol, and 0.001-10 mg/mL of the RNA ligase. Preferably, a catalytic temperature of the RNA ligase is 4-50 °C, including, but not limited to, 4, 10, 15, 20, 25, 30, 35, 40, 45, or 50 °C; and a catalytic time is 0.1-24 h, including, but not limited to, 0.1, 0.5, 1, 2, 3, 4, 5, 7, 10, 15, 20, or 24 h. Preferably, the divalent metal ions include, but are not limited to, magnesium ions, and are derived from reagents such as MgCl₂ or MgSO₄, etc.

The concentration of the above substrate includes, but is not limited to, 1, 5, 10, 20, 50, 100, 200, 500, 1000, 2000, 3000, 4000, 5000, 8000, 10000, 15000, or 20000 µM. The concentration of the ATP includes, but is not limited to, 1, 10, 20, 50, 100, 200, 500, 1000, 1500, 2000, 3000, 4000, 5000, 8000, 10000, 20000, or 40000 µM. The concentration of the RNA ligase includes, but is not limited to, 0.001, 0.01, 0.02, 0.05, 0.1, 0.2, 0.5, 1, 1.5, 2, 5, or 10 mg/mL.

In a preferred embodiment, the substrate includes one or more non-natural nucleotides, and the RNA containing the phosphorothioate bond includes a non-natural RNA containing a phosphorothioate bond. Preferably, the number of the non-natural nucleotides in the substrate is ≥2. Preferably, the 5' terminal and/or 3' terminal of the substrate are the non-natural nucleotides. Preferably, the substrate consists of the non-natural nucleotides. Preferably, the non-natural nucleotides include ribonucleotide having one or more of the following modifications: a ribose position 2' modification, a ribose backbone modification, a base modification or a phosphate backbone modification. Preferably, the ribose position 2' modification includes a 2'-methoxy modification, a 2'-fluoro modification, a 2'-hydrogen modification, a 2'-methoxyethyl modification, a 2'-FANA modification, a locked nucleic acid modification or a hexitol nucleic acid modification. Preferably, the ribose backbone modification includes replacing a ribose in the nucleotide with ribuloNA, TNA, tPhoNA, or dXNA. Preferably, the base modification includes a deazaadenine C7 modification, a deazaguanosine C7 modification, a cytosine C5 modification, or a uridine C5 modification. Preferably, the phosphate backbone modification includes a PS modification.

The locked nucleic acid modification is 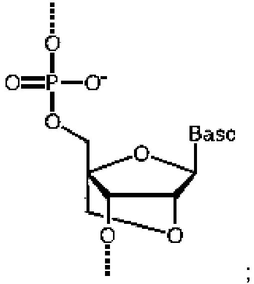 the hexitol nucleic acid modification is 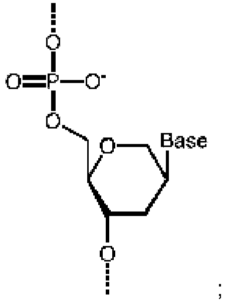 2' methoxyethyl modification is 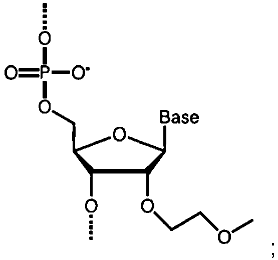 2' methoxy modification is 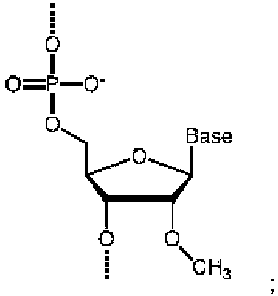 2' fluoro modification is 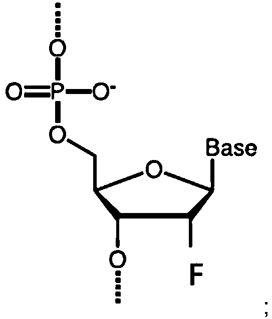 2'-FANA is 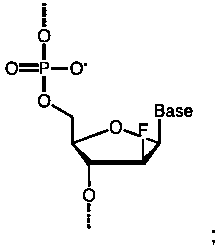 ribuloNA is 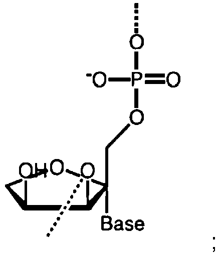 TNA is 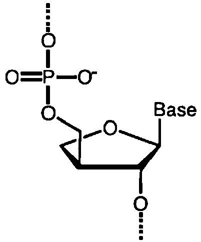 tPhoNA is 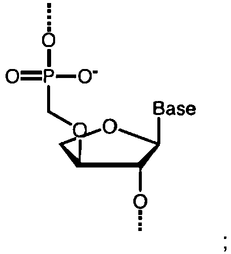 dXNA is 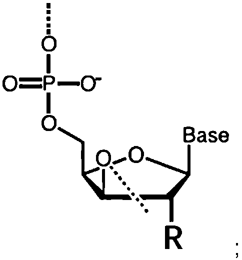 and PS modification is 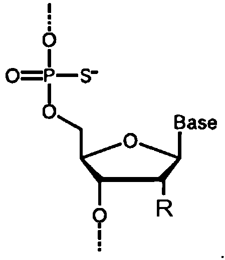 Bases in the above structures all represent bases. For the above modification of non-natural nucleotides, refer to literature: Duffy K, Arangundy-Franklin S, Holliger P. Modified nucleic acids: replication, evolution, and next-generation therapeutics [J].BMC Biology, 2020, 18(1):112. Xeno-nucleic acids (XNA) are a class of nucleic acid molecules having non-natural backbones or nucleic acid bases. Non-natural ribonucleotide is ribonucleotide having a non-natural backbone or nucleic acid base. The above substrate contains the non-natural ribonucleotide, and the RNA prepared through ligation contains the non-natural ribonucleotide, belonging to the non-natural RNA. When the 3' terminal base of a substrate donor and/or the 5' terminal base of a substrate receptor are non-natural bases, although a base at a junction is different from a natural base and belongs to an artificially-created non-natural base, the above RNA ligase can also achieve ligation activity.

In the non-natural ribonucleotide, the 2' modification increases 2' steric hindrance, such as methoxy, or changes the property of the 2' group, such as fluoro. The preparation method disclosed in the present application can ligate 2' modified nucleic acids, such that the natural nucleic acids that serve as natural substrates of the ligases and have 2' hydroxyl can also be ligated with the above preparation method.

In a second typical implementation of the present application, an RNA containing a phosphorothioate bond is provided. The RNA containing the phosphorothioate bond includes the RNA containing the phosphorothioate bond obtained through preparation using the above preparation method.

The beneficial effects of the present application are further described in detail below with reference to specific embodiments.

### Embodiment 1: Ligation of single-stranded RNA containing thio modification

Two single-stranded non-natural RNA substrates F1 and sF2 were designed in this embodiment. The length of the F1 was 13 nt, 5' was hydroxyl, and 3' was hydroxyl; and the sF2 was 10 nt, with a 5' phosphorothioate modification, and the 3' terminal was sealed by a 3' protecting group.
F1: SEQ ID NO: 23: UmCmCmUmUmAmGfUmCfAfGfUmAm.
sF2: SEQ ID NO: 24: GmAmUmAmUmUmUmUmAmUm.

The m after A, C, G, or U represented 2' methoxy modification for the ribonucleotide, and the f represented 2' fluoro modification for the ribonucleotide.

The ribonucleotides at positions 1, 2, 3, 4, 5, 6, 8, 12, and 13 of the F1 had 2' methoxy modifications, and the ribonucleotides at positions 7, 9, 10, and 11 had 2' fluoro modifications.

The ribonucleotides at positions 1, 2, 3, 4, 5, 6, 7, 8, 9, and 10 of the sF2 had 2' methoxy modifications.

The 3' hydroxyl of the F1 and the 5' phosphorothioate of the sF2 formed the phosphorothioate bond under the catalysis of an RNA ligase, so as to form a piece of single-stranded RNA with 23 nt through ligation.

A ligation reaction was performed with the RNA ligase, a ligation reaction system including 50 mM Tris-HCl (pH 7.5), 10 mM MgCl₂, 1 mM DTT, 400 µM ATP, 200 µM F1, and 200 µM sF2 was prepared, the reaction was performed for 16 h at 16 °C, and after the reaction completed, incubation was performed for 5 min at 80 °C to terminate the reaction.

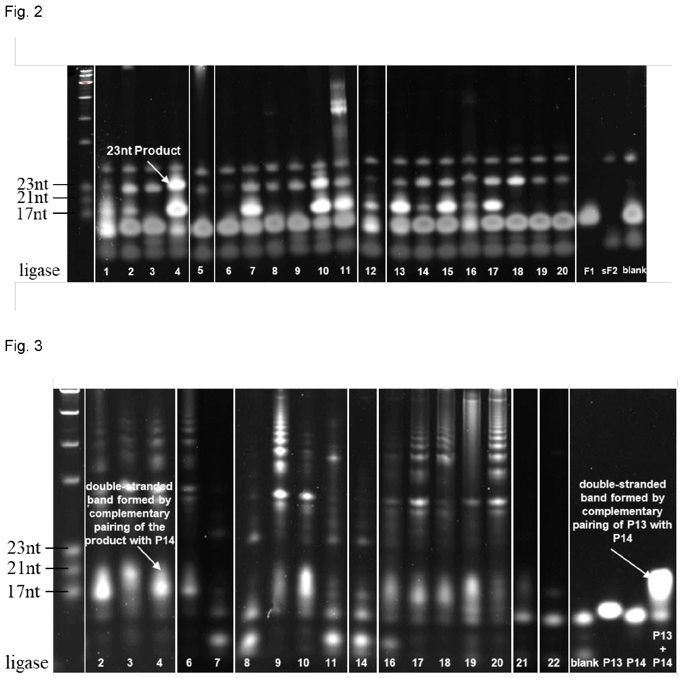

The reaction system was detected with urea-denatured polyacrylamide gel electrophoresis. Gel electrophoresis detection results were shown in Fig. 2, blank was a negative control group without adding the RNA ligase. Compared with the blank, new RNA bands were detected in the reaction system of the RNA ligases ligase-1(SEQ ID NO: 1), ligase-2(SEQ ID NO: 2), ligase-3(SEQ ID NO: 3), ligase-4(SEQ ID NO: 4), ligase-5(SEQ ID NO: 5), ligase-6(SEQ ID NO: 6), ligase-7(SEQ ID NO: 7), ligase-8(SEQ ID NO: 8), ligase-9(SEQ ID NO: 9), ligase-10(SEQ ID NO: 10), ligase-11(SEQ ID NO: 11), ligase-12(SEQ ID NO: 12), ligase-13(SEQ ID NO: 13), ligase-14(SEQ ID NO: 14), ligase-15(SEQ ID NO: 15), ligase-16(SEQ ID NO: 16), ligase-17(SEQ ID NO: 17), ligase-18(SEQ ID NO: 18), ligase-19(SEQ ID NO: 19), and ligase-20(SEQ ID NO: 20) between 21 nt and 25 nt. The molecular weights of the new bands detected through liquid chromatography-mass spectrometry analysis were consistent with the theoretical molecular weight of the ligation product of F1 and sF2.

No ligation product band was detected in the reaction system of the RNA ligases ligase-23(*Methanothermobacter thermautotrophicus*, RNA ligase 3, NCBI Reference Sequence: WP_048060995.1), ligase-24(*Naegleria gruberi*, RNA ligase 5, NCBI Reference Sequence: XP_002669268.1), ligase-25(*Pyrococcus horikoshii*, RNA ligase RtcB, PDB: 4DWR_A), ligase-26(*Thermococcus barophilus*, RNA ligase 1, NCBI Reference Sequence: WP_013466453.1), ligase-27(*Cronobacter phage*, RNA ligase 2, NCBI Reference Sequence: YP_003858534.1), indicating that only specific wild-type RNA ligase might catalyze the ligation reaction of the single-stranded RNA containing the thio modification.

The catalytic activity of the RNA ligase through UPLC detection analysis was shown in Table 1.

**Table 1**

| RNA ligase name | Sequence No. | F1+sF2 catalytic activity |
|---|---|---|
| ligase-1 | SEQ ID NO: 1 | + |
| ligase-2 | SEQ ID NO: 2 | +++ |
| ligase-3 | SEQ ID NO: 3 | + |
| ligase-4 | SEQ ID NO: 4 | ++++ |
| ligase-5 | SEQ ID NO: 5 | + |
| ligase-6 | SEQ ID NO: 6 | + |
| ligase-7 | SEQ ID NO: 7 | +++ |
| ligase-8 | SEQ ID NO: 8 | + |
| ligase-9 | SEQ ID NO: 9 | +++ |
| ligase-10 | SEQ ID NO: 10 | ++++ |
| ligase-11 | SEQ ID NO: 11 | ++++ |
| ligase-12 | SEQ ID NO: 12 | + |
| ligase-13 | SEQ ID NO: 13 | + |
| ligase-14 | SEQ ID NO: 14 | ++++ |
| ligase-15 | SEQ ID NO: 15 | +++ |
| ligase-16 | SEQ ID NO: 16 | + |
| ligase-17 | SEQ ID NO: 17 | +++ |
| ligase-18 | SEQ ID NO: 18 | ++++ |
| ligase-19 | SEQ ID NO: 19 | ++ |
| ligase-20 | SEQ ID NO: 20 | + |

| | | |
|---|---|---|
| Note: "+" indicated 0-1% (excluding an endpoint value of 0%) purity of the product system; "++" indicated 1-2% (excluding an endpoint value of 1%) purity of the product system; "+++" indicated 2-3% (excluding an endpoint value of 2%) purity of the product system; and "++++" indicated 3-4% (excluding an endpoint value of 3%) purity of the product system. In the present application, "the purity of the product system" indicated that in the reaction product, a ratio of a target product to a total component (target product + remaining substrate) in the reaction system might be calculated by the area under the curve of different components in liquid chromatography results. | | |

### Embodiment 2: Ligation of single-stranded RNA containing thio modification (splint ligation method)

Two single-stranded non-natural RNA substrates P11 and P15 and a splint P14 were designed in this embodiment. The length of the P11 was 9 nt, and 3' was hydroxyl; the P15 was 6 nt, with a 5' phosphorothioate modification; the P14 was an RNA sequence with 15 nt; and the P13 was single-stranded RNA with 15 nt without a thio modification and complementarily paired with the P14.
P11: AmCfGmGfGmGfUmCfAm.
P15: CfUmGfAmGfUm.
P14: SEQ ID NO: 25: AmCfUmCfAmGfUmGfAmCfCmCfCmGfUm.
P13: SEQ ID NO: 26: AmCfGmGfGmGfUmCfAmCfUmGfAmGfUm.

The m after A, C, G, or U represented 2' methoxy modification for the RNA, and the f represented 2' fluoro modification for the ribonucleotide.

The ribonucleotides at positions 1, 3, 5, 7, and 9 of the P11 had 2' methoxy modifications, and the ribonucleotides at positions 2, 4, 6, and 8 had 2' fluoro modifications.

The ribonucleotides at positions 2, 4, and 6 of the P15 had 2' methoxy modifications, and the ribonucleotides at positions 1, 3, and 5 had 2' fluoro modifications.

The ribonucleotides at positions 1, 3, 5, 7, 9, 11, 13, and 15 of the P14 had 2' methoxy modifications, and the ribonucleotides at positions 2, 4, 6, 8, 10, 12, and 14 had 2' fluoro modifications.

The ribonucleotides at positions 1, 3, 5, 7, 9, 11, 13, and 15 of the P13 had 2' methoxy modifications, and the ribonucleotides at positions 2, 4, 6, 8, 10, 12, and 14 had 2' fluoro modifications.

The P11 and P15 annealed with the splint P14 to form a complementarily-paired double-stranded structure with a nick. Under the catalysis of the RNA ligase, the 3' hydroxyl of the P11 and the 5' phosphorothioate of the P15 formed the phosphorothioate bond, so as to form a piece of RNA with 15 nt through ligation.

A ligation reaction was performed with the RNA ligase, a ligation reaction system including 50 mM Tris-HCl (pH 7.5), 10 mM MgCl₂, 1 mM DTT, 1 mM ATP, 20 µM P11, 20 µM P15, and 20 µM P14 was prepared, the reaction was performed for 16 h at 16 °C, and after the reaction completed, incubation was performed for 5 min at 80 °C to terminate the reaction.

The reaction system was detected with urea-denatured polyacrylamide gel electrophoresis. Gel electrophoresis detection results were shown in Fig. 3, blank was a negative control group without adding the RNA ligase. Compared with the blank, new RNA bands were detected in the reaction system of the RNA ligases ligase-2(SEQ ID NO: 2), ligase-3(SEQ ID NO: 3), ligase-4(SEQ ID NO: 4), ligase-6(SEQ ID NO: 6), ligase-7(SEQ ID NO: 7), ligase-8(SEQ ID NO: 8), ligase-9(SEQ ID NO: 9), ligase-10(SEQ ID NO: 10), ligase-11(SEQ ID NO: 11), ligase-14(SEQ ID NO: 14), ligase-16(SEQ ID NO: 16), ligase-17(SEQ ID NO: 17), ligase-18(SEQ ID NO: 18), ligase-19(SEQ ID NO: 19), ligase-20(SEQ ID NO: 20), ligase-21(SEQ ID NO: 21), and ligase-22(SEQ ID NO: 22) at corresponding positions of P13+P14 standard products. Mass spectrometry analysis detected new molecular weights in the reaction system that were consistent with the theoretical molecular weight of the the ligation product of P11 and P15.

No ligation product band was detected in the reaction system of the RNA ligases ligase-23(*Methanothermobacter thermautotrophicus*, RNA ligase 3, NCBI Reference Sequence: WP_048060995.1), ligase-24(*Naegleria gruberi*, RNA ligase 5, NCBI Reference Sequence: XP_002669268.1), ligase-25(*Pyrococcus horikoshii*, RNA ligase RtcB, PDB: 4DWR_A), ligase-26(*Thermococcus barophilus*, RNA ligase 1, NCBI Reference Sequence: WP_013466453.1), ligase-27(*Cronobacter phage*, RNA ligase 2, NCBI Reference Sequence: YP_003858534.1), indicating that only specific wild-type RNA ligase could catalyze the splint method ligation reaction of the single-stranded RNA containing the thio modification.

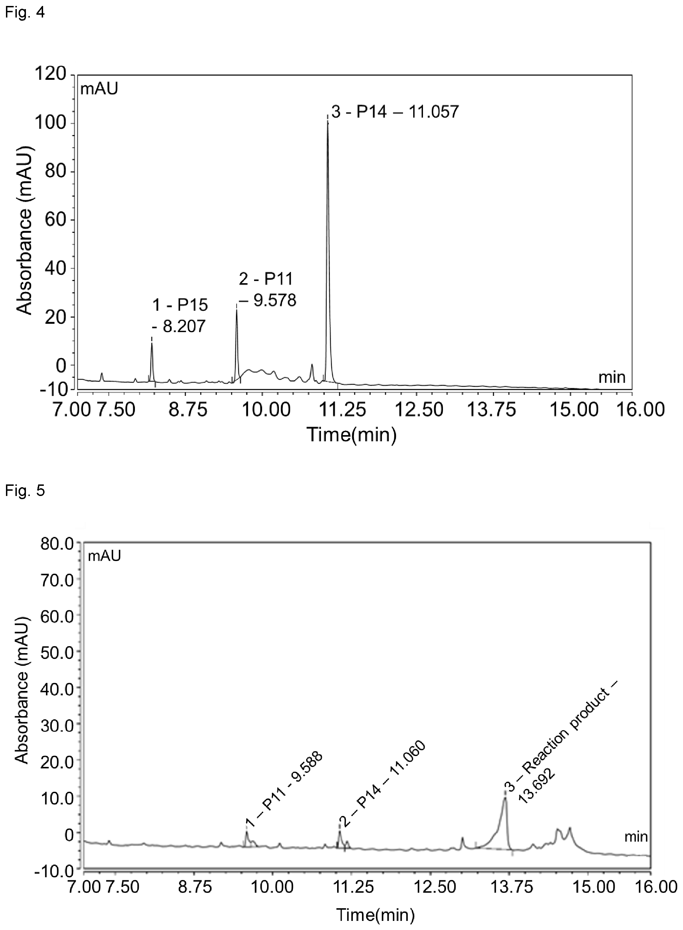

A UPLC detection spectrum before the reaction was shown in Fig. 4, and a spectrum after the reaction was shown in Fig. 5. Through comparison, it might be learned that, a new reaction product peak was formed after the splint P14 was complementarily paired with the ligation product. The catalytic activity of the RNA ligase through UPLC analysis was shown in Table 2.

**Table 2**

| Ligase name | Sequence No. | P11+P15 catalytic activity |
|---|---|---|
| ligase-2 | SEQ ID NO: 2 | ++++ |
| ligase-3 | SEQ ID NO: 3 | + |
| ligase-4 | SEQ ID NO: 4 | +++ |
| ligase-6 | SEQ ID NO: 6 | + |
| ligase-7 | SEQ ID NO: 7 | + |
| ligase-8 | SEQ ID NO: 8 | + |
| ligase-9 | SEQ ID NO: 9 | ++ |
| ligase-10 | SEQ ID NO: 10 | ++++ |
| ligase-11 | SEQ ID NO: 11 | + |
| ligase-14 | SEQ ID NO: 14 | ++ |
| ligase-16 | SEQ ID NO: 16 | ++ |
| ligase-17 | SEQ ID NO: 17 | +++ |
| ligase-18 | SEQ ID NO: 18 | ++++ |
| ligase-19 | SEQ ID NO: 19 | ++++ |
| ligase-20 | SEQ ID NO: 20 | + |
| ligase-21 | SEQ ID NO: 21 | + |
| ligase-22 | SEQ ID NO: 22 | + |

| | | |
|---|---|---|
| Note: "+" indicated 0-10% (excluding an endpoint value of 0%) purity of the product system; "++" indicated 10-20% (excluding an endpoint value of 10%) purity of the product system; "+++" indicated 20-30% (excluding an endpoint value of 20%) purity of the product system; and "++++" indicated 30-40% (excluding an endpoint value of 30%) purity of the product system. | | |

### Embodiment 3: Ligation of single-stranded RNA containing thio modification (splint ligation method)

Two single-stranded non-natural RNA substrates SN-1 and SN-2 and a DNA splint Nus-R were designed in this embodiment. The length of the SN-1 was 8 nt, 5' was hydroxyl, and 3' was hydroxyl; and the SN-2 was 10 nt, with a 5' phosphorothioate modification, and the 3' was a 3' protecting group; and the Nus-R was a DNA sequence of 16 nt.
SN-1: UmsCmsAmsCmsUmsUmsUmsCms.
SN-2: SEQ ID NO: 27: AmsUmsAmsAmsUmsGmsCmsUmsGmsGms.
Nus-R: SEQ ID NO: 28: dAdGdCdAdTdTdAdTdGdAdAdAdGdTdGdA.

The m after A, C, G, or U represented 2' methoxy modification for the ribonucleotide, the s represented the 5' phosphorothioate modification for the RNA, and the d before A, C, G, or U represented that the nucleotide was DNA.

The ribonucleotides at positions 1, 2, 3, 4, 5, 6, 7, and 8 of the SN-1 had the 2' methoxy modifications, and the ribonucleotides at positions 1, 2, 3, 4, 5, 6, 7, and 8 had 5' phosphorothioate modifications.

The ribonucleotides at positions 1, 2, 3, 4, 5, 6, 7, 8, 9, and 10 of the SN-2 had the 2' methoxy modifications, and the ribonucleotides at positions 1, 2, 3, 4, 5, 6, 7, 8, 9, and 10 had the 5' phosphorothioate modifications.

The nucleotides at positions 1-16 of the Nus-R were all deoxyribonucleotides (DNA).

A ligation reaction was performed with the following RNA ligase, a ligation reaction system including 50 mM Tris-HCl (pH 7.5), 10 mM MgCl₂, 1 mM DTT, 1 mM ATP, 100 µM SN-1, 100 µM SN-2, 100 µM Nus-R, and 0.2 mg/mL ligase was prepared, the reaction was performed for 16 h at 16 °C, and after the reaction completed, incubation was performed for 5 min at 80 °C to terminate the reaction.

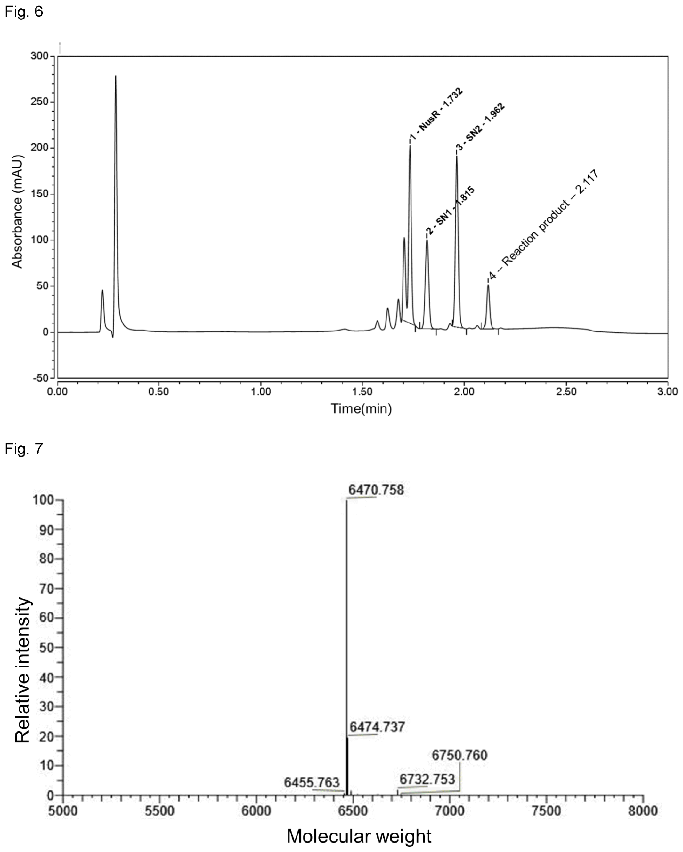

UPLC detection results were shown in Fig. 6. New peaks were detected in the reaction system of the RNA ligases ligase-2(SEQ ID NO: 2), ligase-3(SEQ ID NO: 3), ligase-4(SEQ ID NO: 4), ligase-5(SEQ ID NO: 5), ligase-6(SEQ ID NO: 6), ligase-9(SEQ ID NO: 9), ligase-10(SEQ ID NO: 10), ligase-11(SEQ ID NO: 11), ligase-14(SEQ ID NO: 14), ligase-17(SEQ ID NO: 17), ligase-18(SEQ ID NO: 18), ligase-19(SEQ ID NO: 19), ligase-20(SEQ ID NO: 20), and ligase-22(SEQ ID NO: 22). The new peaks were identified using liquid chromatography-mass spectrometry, results were shown in Fig. 7, and the molecular weight of the new peaks was detected to be consistent with the theoretical molecular weight of the ligation product of SN-1 and SN-2.

### Embodiment 4: Ligation of single-stranded RNA containing thio modification

Two single-stranded non-natural RNA substrates SN-1 and SN-2 were designed in this embodiment. The length of the SN-1 was 8 nt, 5' was hydroxyl, and 3' was hydroxyl; and the SN-2 was 10 nt, with a 5' phosphorothioate modification, and the 3' was a 3' protecting group.
SN-1: UmsCmsAmsCmsUmsUmsUmsCms.
SN-2: SEQ ID NO: 27: AmsUmsAmsAmsUmsGmsCmsUmsGmsGms.

The m after A, C, G, or U represented the 2' methoxy modification for the RNA, and the s represented the 5' phosphorothioate modification for the RNA.

The ribonucleotides at positions 1, 2, 3, 4, 5, 6, 7, and 8 of the SN-1 had 2' methoxy modifications, and the ribonucleotides at positions 1, 2, 3, 4, 5, 6, 7, and 8 had 5' phosphorothioate modifications.

The ribonucleotides at positions 1, 2, 3, 4, 5, 6, 7, 8, 9, and 10 of the SN-2 had 2' methoxy modifications, and the ribonucleotides at positions 1, 2, 3, 4, 5, 6, 7, 8, 9, and 10 had 5' phosphorothioate modifications.

A ligation reaction was performed with the RNA ligase, a ligation reaction system including 50 mM Tris-HCl (pH 7.5), 10 mM MgCl₂, 1 mM DTT, 1 mM ATP, 100 µM SN-1, 100 µM SN-2, and 0.2 mg/mL ligase was prepared, the reaction was performed for 16 h at 16 °C, and after the reaction completed, incubation was performed for 5 min at 80 °C to terminate the reaction.

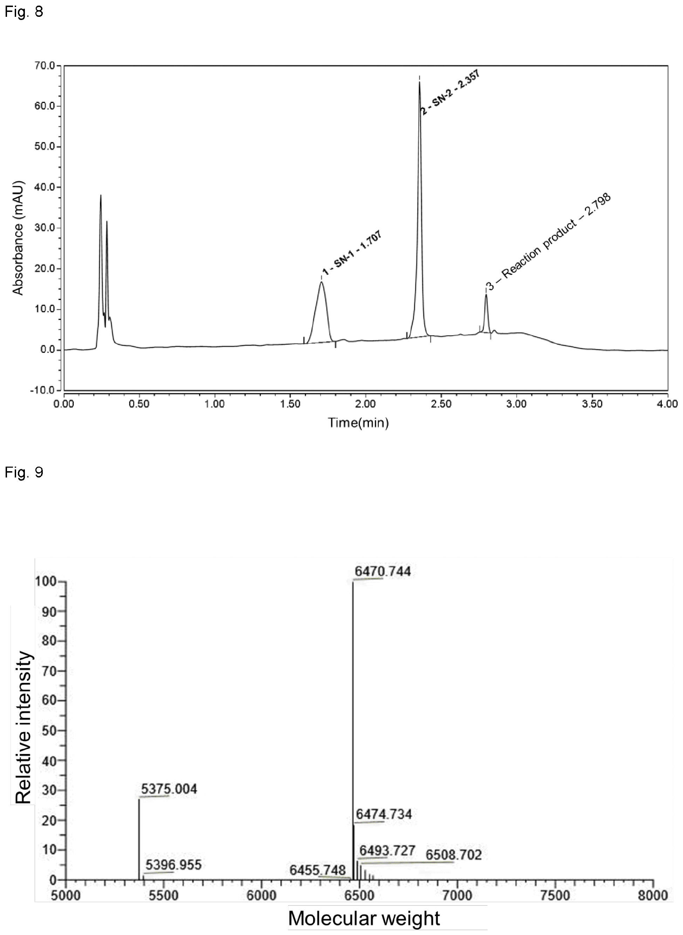

UPLC detection results were shown in Fig. 8. New peaks were detected in the reaction system of the RNA ligases ligase-1(SEQ ID NO: 1), ligase-4(SEQ ID NO: 4), ligase-7(SEQ ID NO: 7), ligase-8(SEQ ID NO: 8), ligase-9(SEQ ID NO: 9), ligase-10(SEQ ID NO: 10), ligase-11(SEQ ID NO: 11), ligase-12(SEQ ID NO: 12), ligase-13(SEQ ID NO: 13), ligase-14(SEQ ID NO: 14), ligase-15(SEQ ID NO: 15), ligase-16(SEQ ID NO: 16), ligase-18(SEQ ID NO: 18), and ligase-19(SEQ ID NO: 19). The new peaks were identified using liquid chromatography-mass spectrometry, results were shown in Fig. 9, and the molecular weight of the new peaks was detected to be consistent with the theoretical molecular weight of the ligation product of SN-1 and SN-2.

### Embodiment 5: Ligation of double-stranded RNA containing thio modification

Four pieces of single-stranded non-natural RNA were designed in this embodiment, where the length of d1 was 12 nt, 5' was hydroxyl, and 3' was hydroxyl; the length of d2 was 9 nt, with a 5' phosphorothioate modification, and 3' was a 3' protecting group; the length of d3 was 9 nt, with a 5' phosphorothioate modification; and the length of d4 was 14 nt, and 3' was hydroxyl. The d1 and the d3 could be complementarily paired with each other to form double-stranded DNA, the d2 and the d4 could be complementarily paired with each other to form double-stranded DNA, and the above two pieces of double-stranded DNA might serve as two substrates for double-stranded RNA ligation.
d1: SEQ ID NO: 29: UmAmsCmsUmCmUmGfAmGfUfCfUm.
d2: AmsUmUmGmAmUmAmUmsUms.
d3: CmsAfGmAmGmUmAmUmsAms.
d4: SEQ ID NO: 30: AmAfsUmsAmUmCfAmAmUmAmGmAmCmUf.

The m after A, C, G, or U represented 2' methoxy modification for the RNA, the f represented 2' fluoro modification for the RNA, and the s represented 5' phosphorothioate modification for the RNA.

The ribonucleotides at positions 1, 2, 3, 4, 5, 6, 8, and 12 of the d1 had 2' methoxy modifications, the ribonucleotides at positions 7, 9, 10, and 11 had 2' fluoro modifications, and the ribonucleotides at positions 2 and 3 had 5' phosphorothioate modifications.

The ribonucleotides at positions 1, 2, 3, 4, 5, 6, 7, 8, and 9 of the d2 had 2' methoxy modifications, and the ribonucleotides at positions 1, 8, and 9 had 5' phosphorothioate modifications.

The ribonucleotides at positions 1, 3, 4, 5, 6, 7, 8, and 9 of the d3 had 2' methoxy modifications, the ribonucleotide at position 2 had the 2' fluoro modification, and the ribonucleotides at positions 1, 8 and 9 had 5' phosphorothioate modifications.

The ribonucleotides at positions 1, 3, 4, 5, 7, 8, 9, 10, 11, 12, and 13 of the d4 had 2' methoxy modifications, the ribonucleotides at positions 2, 6, and 14 had 2' fluoro modifications, and the ribonucleotides at positions 2 and 3 had 5' phosphorothioate modifications.

A ligation reaction was performed with the RNA ligase, a ligation reaction system including 50 mM Tris-HCl (pH 7.5), 10 mM MgCl₂, 1 mM DTT, 1 mM ATP, 50 µM d1, 50 µM d2, 50 µM d3, 50 µM d4, and 0.2 mg/mL ligase was prepared, the reaction was performed for 16 h at 16 °C, and after the reaction completed, incubation was performed for 5 min at 80 °C to terminate the reaction.

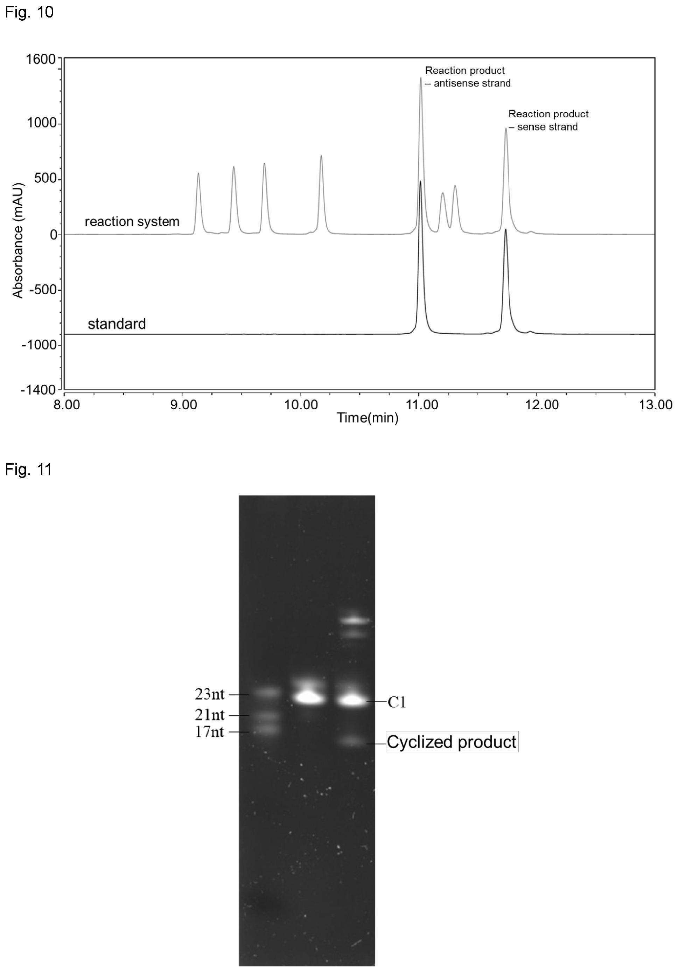

UPLC detection results were shown in Fig. 10. Two new peaks corresponding to a standard product were detected in the reaction system of the RNA ligases ligase-2(SEQ ID NO: 2), ligase-3(SEQ ID NO: 3), ligase-4(SEQ ID NO: 4), ligase-5(SEQ ID NO: 5), ligase-6(SEQ ID NO: 6), ligase-9(SEQ ID NO: 9), ligase-10(SEQ ID NO: 10), ligase-11(SEQ ID NO: 11), ligase-14(SEQ ID NO: 14), ligase-17(SEQ ID NO: 17), ligase-18(SEQ ID NO: 18), ligase-19(SEQ ID NO: 19), ligase-20(SEQ ID NO: 20), and ligase-22(SEQ ID NO: 22). Mass spectrometry identification was performed on the two product peaks, and had the molecular weight consistent with the theoretical molecular weight of the ligation product.

### Embodiment 6: Cyclization of single-stranded RNA containing thio modification

One single-stranded non-natural RNA substrate C1 was designed in this embodiment, which had a length being 24 nt and had a 5' phosphorothioate modification and a 3' hydroxyl.

C1: SEQ ID NO: 31: GmsAmCmUmGmAmCmAmGmAmUmGmGmCmCmUmAmUmAmAmCmGmUmGm.

The m after A, C, G, or U represented the 2' methoxy modification for the RNA, and the s represented the 5' phosphorothioate modification for the RNA.

The ribonucleotides at positions 1, 2, 3, 4, 5, 6, 7, 8, 9, 10, 11, 12, 13, 14, 15, 16, 17, 18, 19, 20, 21, 22, 23, and 24 of the C1 had 2' methoxy modifications, and the ribonucleotide at position 1 had 5' phosphorothioate modification.

A cyclization reaction was performed with the RNA ligase, a ligation reaction system including 50 mM Tris-HCl (pH 7.5), 10 mM MgCl₂, 1 mM DTT, 20 µM ATP, 10 µM C1, and 0.2 mg/mL ligase was prepared, and the reaction was performed for 16 h at 37 °C.

Product molecular weights were detected in the reaction system of the RNA ligases ligase-1(SEQ ID NO: 1), ligase-4(SEQ ID NO: 4), ligase-7(SEQ ID NO: 7), ligase-8(SEQ ID NO: 8), ligase-9(SEQ ID NO: 9), ligase-10(SEQ ID NO: 10), ligase-11(SEQ ID NO: 11), ligase-12(SEQ ID NO: 12), ligase-13(SEQ ID NO: 13), ligase-14(SEQ ID NO: 14), ligase-15(SEQ ID NO: 15), ligase-16(SEQ ID NO: 16), ligase-18(SEQ ID NO: 18), and ligase-19(SEQ ID NO: 19) through mass spectrometry analysis. Bands in the reaction system were analyzed using urea-denatured polyacrylamide gel electrophoresis. As shown in Fig. 11, a new band, which was a cyclized product band, was detected under a C1 band.

The main method for synthesizing RNA at present was solid-phase synthesis. The RNA required for the synthesis of targets required a plurality of rounds of accumulation. Furthermore, during the synthesis of an RNA strand, yield decreased as chain length increased, and impurities in the obtained product also rose, leading to a cumbersome purification process, and thus significantly increasing costs. In view of the various shortcomings of solid-phase synthesis, numerous new methods for synthesizing RNA were under study, and one is to use an RNA ligase to generate RNA through a ligation reaction. The RNA ligases were a class of enzymes capable of ligating short RNA fragments. This method offered advantages such as high efficiency, low cost, environmental friendliness, etc.

It may be seen from the above description that, in the above embodiments of the present disclosure, the following technical effects are realized. The present application develops a method for preparing a RNA containing a phosphorothioate bond. The 3' hydroxyl of the substrate is ligated to the 5' phosphorothioate with the above preparation method, and the RNA containing the phosphorothioate bond that is difficultly obtained in the prior art can be prepared.

The above are only the preferred embodiments of the present disclosure and are not intended to limit the present disclosure. For person skilled in the art, the present disclosure may have various modifications and variations. Any modifications, equivalent replacements, improvements and the like made within the spirit and principle of the present disclosure all fall within the scope of protection of the present disclosure.

## Claims

1. A preparation method for an RNA comprising a phosphorothioate bond, wherein the preparation method comprises:
catalyzing the ligation of a 3' hydroxyl to a 5' phosphorothioate of a substrate with an RNA ligase, so as to form the RNA comprising the phosphorothioate bond; wherein
the 5' phosphorothioate comprises 5' phosphate comprising a thio modification, and the thio modification comprises the replacement of an oxygen atom in P-OH or P=O of the 5' phosphate by a sulfur atom, thereby correspondingly forming P-SH or P=S; and
the RNA ligase comprises any one or more enzymes of RNA ligase families 1, 2 or 3.

2. The preparation method of claim 1, wherein the RNA ligase comprises one or more of RNA ligases as shown in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21 or SEQ ID NO: 22; or
an enzyme having 70% or more identity to any one of the RNA ligases as shown in SEQ ID NO: 1 to SEQ ID NO: 22, and having an activity of catalyzing the ligation of the 3' hydroxyl and the 5' phosphorothioate.

3. The preparation method of claim 1, wherein the substrate comprises a single-stranded RNA or a double-stranded RNA, and correspondingly, the RNA comprising the phosphorothioate bond comprises a single-stranded RNA comprising a phosphorothioate bond or a double-stranded RNA comprising a phosphorothioate bond; and
the number of the substrate is one, two or more.

4. The preparation method of claim 3, wherein when the number of the substrate is 1 and the substrate is the single-stranded RNA, the 5' terminal of the substrate is the 5' phosphorothioate, and the 3' terminal of the substrate is the 3' hydroxyl,
the 5' terminal of the substrate and the 3' terminal of the substrate are ligated and cyclized under the catalysis of the RNA ligase, so as to obtain a cyclic single-stranded RNA comprising a phosphorothioate bond.

5. The preparation method of claim 4, wherein the cyclization of the single-stranded RNA is guided using a splint, the splint specifically binds to at least three bases from the 3' terminal to the 5' terminal of the single-stranded RNA, and the splint specifically binds to at least three bases from the 5' terminal to the 3' terminal of the single-stranded RNA, and bases of the splint which specifically bind to the 3' terminal of the single-stranded RNA are adjacent to bases which specifically bind to the 5' terminal of the single-stranded RNA.

6. The preparation method of claim 3, wherein when the number of the substrate is 2 and the substrates are the single-stranded RNA, the substrates comprise a first substrate and a second substrate, the 3' terminal of the first substrate is the 3' hydroxyl, and the 5' terminal of the second substrate is the 5' phosphorothioate, and
the 3' terminal of the first substrate and the 5' terminal of the second substrate are ligated under the catalysis of the RNA ligase to obtain a linear single-stranded RNA comprising a phosphorothioate bond.

7. The preparation method of claim 6, wherein the 3' terminal of the second substrate is a 3' terminal protecting group, and the 5' terminal of the first substrate is a 5' terminal protecting group.

8. The preparation method of claim 3, wherein when the number of the substrate is 2 and the substrates are the double-stranded RNA, the substrates comprise a first substrate and a second substrate, and each substrate comprises a sense strand and an antisense strand, and;
the 3' terminal of the sense strand of the first substrate is the 3' hydroxyl, and the 5' terminal of the antisense strand of the first substrate is the 5' phosphate;
the 5' terminal of the sense strand of the second substrate is the 5' phosphate, and the 3' terminal of the antisense strand of the second substrate is the 3' hydroxyl;
at least one of the 5' phosphate of the antisense strand of the first substrate and the sense strand of the second substrate is the 5' phosphorothioate;
the 3' terminal of the sense strand of the first substrate and the 5' terminal of the sense strand of the second substrate are ligated under the catalysis of the RNA ligase; and the 5' terminal of the antisense strand of the first substrate and the 3' terminal of the antisense strand of the second substrate are ligated under the catalysis of the RNA ligase;
so as to obtain a linear double-stranded RNA comprising a phosphorothioate bond.

9. The preparation method of claim 8, wherein the 3' terminal of the antisense strand of the first substrate and the 3' terminal of the sense strand of the second substrate are both 3' terminal protecting groups; and
the 5' terminal of the sense strand of the first substrate and the 5' terminal of the antisense strand of the second substrate are both 5' protecting groups.

10. The preparation method of claim 9, wherein the first substrate and the second substrate are both a double-stranded RNA having different lengths of a sense strand and an antisense strand,
the 3' terminal of the sense strand of the first substrate is complementarily paired with the 3' terminal of the antisense strand of the second substrate, or
the 5' terminal of the antisense strand of the first substrate is complementarily paired with the 5' terminal of the sense strand of the second substrate,
thereby, the 3' terminal of the sense strand of the first substrate is adjacent to the 5' terminal of the sense strand of the second substrate, and the 5' terminal of the antisense strand of the first substrate is adjacent to the 3' terminal of the antisense strand of the second substrate.

11. The preparation method of claim 6, wherein the preparation method further comprises using a splint to guide the ligation between the first substrate and the second substrate;
the splint specifically binds to at least three bases from the 3' terminal to the 5' terminal of the first substrate, and the splint specifically binds to at least three bases from the 5' terminal to the 3' terminal of the second substrate.

12. The preparation method of claim 11, wherein bases of the splint specifically binding to the 3' terminal of the first substrate are adjacent to bases specifically binding to the 5' terminal of the second substrate.

13. The preparation method of any one of claims 6-10, wherein the preparation method is repeatedly used to ligase three or more substrates for multiple times, the number of the substrates ligated each time is two, and the substrates are sequentially ligated to obtain the RNA comprising the phosphorothioate bond.

14. The preparation method of claim 3, wherein the preparation method comprises using a splint to guide the ligation of three or more substrates; and wherein
the splint specifically binds to at least three bases of each of the substrates.

15. The preparation method of claim 14, wherein bases of the substrates specifically binding to the splint are adjacent to each other, and adjacent bases are located at 5' terminal and 3' terminal of different substrates, respectively,
the 5' terminal is 5' phosphate, and at least one of the 5' phosphate is the 5 phosphorothioate; and
the 3' terminal is hydroxyl.

16. The preparation method of claim 1, wherein catalytic system of the RNA ligase comprises 1-20000 µM of the substrate, 1-100000 µM of ATP, 5-100 mM of Tris-HCl, 0.1-20 mM of bivalent metal ions, 0-10 mM of dithiothreitol, and 0.001-10 mg/mL of the RNA ligase.

17. The preparation method of claim 16, wherein a catalytic temperature of the RNA ligase is 4-50 °C, and a catalytic time is 0.1-24 h; and
the divalent metal ions are derived from MgCl₂ or MgSO₄.

18. The preparation method of claim 1, wherein the substrate comprises one or more non-natural nucleotides, and the RNA comprising the phosphorothioate bond comprises a non-natural RNA comprising a phosphorothioate bond.

19. The preparation method of claim 18, wherein the number of the non-natural nucleotides in the substrate is ≥2, and the 5' terminal and/or 3' terminal of the substrate are the non-natural nucleotides.

20. The preparation method of claim 19, wherein the substrate consists of the non-natural nucleotides.

21. The preparation method of any one of claims 18 to 20, wherein,
the non-natural nucleotides comprise a ribonucleotide having one or more of the following modifications: a ribose position 2' modification, a ribose backbone modification, a base modification or a phosphate backbone modification;
the ribose position 2' modification comprises a 2'-methoxy modification, a 2'-fluoro modification, a 2'-hydrogen modification, a 2'-methoxyethyl modification, a 2'-FANA modification, a locked nucleic acid modification, or a hexitol nucleic acid modification;
the ribose backbone modification comprises replacing a ribose in the nucleotide with ribuloNA, TNA, tPhoNA, or dXNA;
the base modification comprises a deazaadenine C7 modification, a deazaguanosine C7 modification, a cytosine C5 modification, or a uridine C5 modification; and
the phosphate backbone modification comprises a PS modification.

22. An RNA comprising a phosphorothioate bond, wherein the RNA comprising the phosphorothioate bond comprises the RNA comprising a phosphorothioate bond prepared and obtained using the preparation method of any one of claims 1 to 21.
